# EUROPÄISCHE PATENTSCHRIFT

(11) **EP 3 612 313 B1**
(45) Veröffentlichungstag und Bekanntmachung des Hinweises auf die Patenterteilung: **12.04.2023**
(21) Anmeldenummer: 18719150.7
(22) Anmeldetag: 19.04.2018
(51) Int. Cl.: B05B 5/00, B05B 5/043, B05B 5/053, B05B 5/16, A61M 35/00, B05B 9/08, A45D 34/02, A45D 34/00, B05B 1/14, B05B 9/04

(54) **ELEKTROSTATISCHER ZERSTÄUBER FÜR FLÜSSIGKEITEN UND VERFAHREN ZUM BETRIEB EINES ELEKTROSTATISCHEN ZERSTÄUBERS**
ELECTROSTATIC ATOMIZER FOR LIQUIDS AND METHOD FOR OPERATING AN ELECTROSTATIC ATOMIZER
ATOMISEUR ÉLECTROSTATIQUE POUR LIQUIDES ET PROCÉDÉ DE FONCTIONNEMENT D'UN ATOMISEUR ÉLECTROSTATIQUE

(30) Priorität: 21.04.2017 DE 102017108610
(43) Veröffentlichungstag der Anmeldung: 26.02.2020
(73) Patentinhaber: J. Wagner GmbH, 88677 Markdorf (DE)
(72) Erfinder: BARTHELMES, Jan, 88682 Salem (DE); GÖHRING, Alfred, 88682 Salem (DE); JELTSCH, Thomas, 88048 Friedrichshafen (DE); STOHL, Holger, 88677 Markdorf (DE)
(74) Vertreter: Otten, Roth, Dobler & Partner mbB Patentanwälte
(86) Internationale Anmeldenummer: PCT/EP2018/060117
(87) Internationale Veröffentlichungsnummer: WO 2018/193065

(56) Entgegenhaltungen:
- EP-A2- 1 388 371
- US-A- 4 467 961
- US-A1- 2003 029 447
- US-A1- 2004 079 360
- US-A1- 2009 200 392
- US-A1- 2010 051 649
- US-A1- 2012 125 950
- US-A1- 2012 248 149

## Beschreibung

Die Erfindung betrifft einen elektrostatischen Zerstäuber für Flüssigkeiten gemäß dem Oberbegriff des Anspruchs 1 sowie ein Verfahren zum Betrieb eines Zerstäubers gemäß dem Oberbegriff des Anspruchs 18.

Elektrostatische Zerstäubung umfasst im Sinne der vorliegenden Erfindung alle Zerstäubungsvorgänge, welche Flüssigkeiten mit Effekten der Einwirkung einer Hochspannung zerstäuben. Insbesondere auch elektrohydrodynamische Effekte sowie elektrokinetische Effekte sind von dem Begriff dieser Art von Zerstäubung umfasst. Im Sinne der Erfindung kann unter einer elektrostatischen Zerstäubung auch eine elektrohydrodynamische Zerstäubung aufgefasst werden.

Aus der US 2010/0116897 A1 ist ein elektrostatischer Zerstäuber für Flüssigkeiten bekannt, welcher ein Gehäuse, eine elektrische Energiequelle, ein Aktivierungsmittel, eine Kontrollelektronik, eine Hochspannungsquelle, einen Flüssigkeitstank, eine Fördervorrichtung und Zerstäuberdüsen umfasst. Eine Förderung der Flüssigkeit erfolgt mittels eines in dem Flüssigkeitstank auf einer Spindel geführten Kolbens.

Aus der US 2004/079360 A1 ist ein Inhalator bekannt, welcher ein Gehäuse umfasst, das eine Kammer enthält, die ein Reservoir für eine Flüssigkeit bereitstellt, die einem Flüssigkeitsauslass zugeführt werden soll. Erste und zweite Elektrode sind voneinander beabstandet, wobei die erste Elektrode am oder neben dem Flüssigkeitsauslass vorgesehen ist. Eine Spannungsversorgung wird als Reaktion auf Luft aktiviert, die durch einen Lufteinlass des Gehäuses strömt, um eine Potentialdifferenz zwischen der ersten und der zweiten Elektrode bereitzustellen.

Aus der EP 1388 371 A2 ist ein Verfahren zum Bereitstellen eines Wundverbandes bekannt, wobei: Flüssigkeit einem Auslass in der Nähe eines Substrats zugeführt wird, auf dem der Wundverband bereitgestellt werden soll, Flüssigkeit, die aus dem Auslass austritt, einem elektrischen Feld ausgesetzt wird, um mindestens einen Strahl elektrisch geladener Flüssigkeit zu bilden, wobei die Flüssigkeit derart ausgebildet ist, dass der mindestens eine Strahl nach der Bildung elektrisch geladenes Fasermaterial bildet, das von dem Substrat angezogen wird und sich auf diesem ablagert, um eine Matte oder eine Bahn darauf zu bilden.

Aus der US 2009 200 392 A1 ist eine Vorrichtung zum Sprühen einer kosmetischen oder dermatologischen Zusammensetzung bekannt, welche einen Ultraschall- oder elektrostatischen Sprühkopf, einen Luftstromgenerator zum Mitreißen eines Sprühens von Zusammensetzungspartikeln, die durch den Sprühkopf zerstäubt werden, und eine Konditionierungsvorrichtung umfassen kann, wobei die Konditionierungsvorrichtung angeordnet ist, um die Temperatur des abgehenden Luftstroms zu modifizieren.

Aus der US 4 467 961 A ist ein Behälter bekannt, der auf einem Fahrzeug befestigbar und für die Aufnahme von flüssigen agrochemischen Formulierungen geeignet ist, mit einem eine Kupplung aufweisenden Mundstück für die Abgabe von Flüssigkeit zu einer Strömungsregeleinrichtung und dann zu mindestens einem Spritzkopf, der von dem Fahrzeug getragen wird, auf welchem der Behälter befestigt werden soll. Hierbei ist der Behälter mit einer Signaleinrichtung versehen, die entsprechend der Natur des vorgesehenen flüssigen Inhalts des Behälters eingestellt ist, wobei die Signaleinrichtung so ausgebildet ist, dass sie die Strömungsregeleinrichtung bei Betätigung derselben und damit die Strömungsgeschwindigkeit beeinflusst.

Aus der US 2012 125 950 A1 ist ein handgehaltener, wiederverwendbarer Produktspender bekannt, der einen Teil des fließfähigen Produkts erwärmt, während es aus einem Reservoir ausgegeben wird. Durch Erhitzen des Produkts können einige Eigenschaften des Produkts verbessert werden. Der Vorratsbehälter ist aus dem wiederverwendbaren Gehäuse abnehmbar.

Aus der US 2003 029 447 A1 ist eine Dosierinhalationsvorrichtung mit einem Hauptgehäuseteil zum Aufnehmen und Abgeben von Inhalationsmedikamenten bekannt.

Aus der US 2012 248 149 A1 ist Flüssigkeitsspender bekannt. In einer beispielhaften Ausführungsform ist der Flüssigkeitsspender konfiguriert, um Schaum abzugeben. Der Flüssigkeitsspender umfasst einen Rahmenabschnitt, einen Pumpabschnitt, einen Nachfüllabschnitt und einen Führungsabschnitt. Der Pumpabschnitt umfasst ein oder mehrere Kompressionselemente, die mit einem drehbaren Element gekoppelt sind. Das drehbare Element ist so konfiguriert, dass es sich relativ zum Rahmenteil dreht. Das Rohr wird durch das eine oder die mehreren Kompressionselemente zusammengedrückt, wenn sich das drehbare Element dreht, um flüssiges Material vom Flüssigkeitsbehälter zur Schaumdüse zu bewegen. Die Schaumdüse ist konfiguriert, um das flüssige Material in Schaum umzuwandeln, der vom Flüssigkeitsspender abgegeben wird.

Schließlich ist aus der US 2010 051 649 A1 ein Spender mit einziehbarer Düse bekannt, der einen Behälter, einen Zylinder, einen Kolben, ein drückendes Element und eine elastische Einheit umfasst.

Der Erfindung liegt die Aufgabe zugrunde, einen elektrostatischen Zerstäuber für Flüssigkeiten bzw. ein Verfahren zum Betrieb eines Zerstäubers vorzuschlagen, bei welchem bzw. durch welches eine alternative Pumpe zum Einsatz kommt.

Diese Aufgabe wird ausgehend von den Merkmalen des Oberbegriffs des Anspruchs 1 bzw. 18 durch die kennzeichnenden Merkmale des Anspruchs 1 bzw. 18 gelöst. In den jeweiligen Unteransprüchen sind vorteilhafte und zweckmäßige Weiterbildungen angegeben.

Die Erfindung ist im beigefügten Anspruchssatz beschrieben.

Bei dem elektrostatischen bzw. elektrohydrodynamischen Zerstäuber für Flüssigkeiten, insbesondere Kosmetika ist die Fördervorrichtung zwischen dem Flüssigkeitstank und den Zerstäuberdüsen angeordnet, wobei die Fördervorrichtung durch eine erste Leitung mit dem Flüssigkeitstank verbunden ist und wobei die Fördervorrichtung durch eine zweite Leitung mit den Zerstäuberdüsen verbunden ist, so dass die Fördervorrichtung Flüssigkeit aus dem Flüssigkeitstank saugt und zu den Zerstäuberdüsen fördert. Durch eine derartige Anordnung der Fördervorrichtung zwischen dem Flüssigkeitstank und den Zerstäuberdüsen bzw. in einem Flüssigkeitslauf ist es möglich, den Flüssigkeitstank als einfach aufgebautes, passives Bauteil auszuführen. Durch die saugende Aufführung der Fördervorrichtung benötigt der Flüssigkeitstank selbst keine druckerzeugenden Bestandteile. Auf diese Weise kann die Flüssigkeit auch ohne jede Belastung des Flüssigkeitstanks an die Zerstäuberdüsen weiter gefördert werden.

Die erste und die zweite Leitung kann dabei bevorzugt einstückig ausgebildet, insbesondere als kostengünstig herstellbarer extrudierter Kunststoffschlauch vorgesehen sein. Derartige Schläuche sind aus dem Bereich der Schlauchpumpen in Form von Peristaltikpumpen bekannt. Erfindungsgemäß ist jedoch bevorzugt vorgesehen, dass pro Zerstäuberdüse ein separater Schlauch von dem Flüssigkeitstank bis zur Zerstäuberdüse führt und insbesondere jeder Schlauch eine Fördervorrichtung, insbesondere eine Schlauchpumpe umfasst oder die Schläuche als Schlauchpaket (z.B. ähnlich eines Flachbandkabels) mit einer Födervorrichtung, insbesondere einer Mehrkanal-Schlauchpumpe die Flüssigkeit zu den jeweiligen Zerstäuberdüsen leiten.

Bezüglich der Ausgestaltungen und der Vorteile der weiteren abhängigen Ansprüche wird auf die Beschreibung der Figuren verwiesen.

Im Sinne der Erfindung kann ein Gitter auch als eine Anordnung mehrerer nebeneinander angeordneter Spalten sowie untereinander angeordneter Zeilen aufgefasst werden.

Alternativ kann im Sinne der Erfindung ein Gitter auch die Eckpunkte eines Dreiecks aufweisen, wie es z.B. beim Kristallgitter der Diamantstruktur gegeben ist.

Im Sinne der Erfindung ist unter einer Flüssigkeit jedwedes Liquid aufzufassen. Im Sinne der Erfindung ist insbesondere vorgesehen, dass die Flüssigkeit ein Kosmetikum, ein Sonnenschutzmittel oder ein Insektenabweisungsmittel ist. Auch eine Anwendung eines Hautfärbemittels für die Bräunung oder Aufhellung der Haut ist denkbar.

Die Flüssigkeit kann aber auch eine flüssige Farbe oder Lack oder dergleichen sein.

Weitere Einzelheiten der Erfindung werden in der Zeichnung anhand von schematisch dargestellten Ausführungsbeispielen beschrieben.

Hierbei zeigt:
- Figur 1:: eine Schnittansicht durch eine schematische Darstellung einer ersten Ausführungsvariante eines Zerstäubers, wobei die Zerstäuberdüsen in einer Ruhestellung stehen;
- Figur 2:: eine Schnittansicht durch die Darstellung der Figur 1, wobei die Zerstäuberdüsen in einer Arbeitsstellung stehen;
- Figur 3:: eine Seitenansicht auf die Figur 1 im Bereich der Zerstäuberdüsen;
- Figur 4a:: eine schematische Seitenansicht einer zweiten Ausführungsvariante eines Zerstäubers;
- Figur 4b:: den in der Figur 4a gezeigten Zerstäuber in einer teleskopierten Stellung;
- Figur 5a: eine schematische Seitenansicht einer dritten Ausführungsvariante eines Zerstäubers;
- Figur 5b:: den in der Figur 5a gezeigten Zerstäuber bei einem gegenüber dem Griffabschnitt um 90° verdrehten Kopfabschnitt;
- Figur 6a: eine schematische Seitenansicht einer vierten Ausführungsvariante eines Zerstäubers und
- Figur 6b:: den in der Figur 6a gezeigten Zerstäuber bei einem gegenüber dem Kopfabschnitt um 45° geknickten Griffabschnitt;
- Figur 7a:: eine alternative Ausführungsform mit drei Zerstäuberdüsen mit Schieber im geschlossenen Zustand;
- Figur 7b:: die alternative Ausführungsform mit drei Zerstäuberdüsen mit Schieber im geöffneten Zustand;
- Figur 7c:: die alternative Ausführungsform unter Darstellung der Baugruppen im Inneren des Gehäuses.

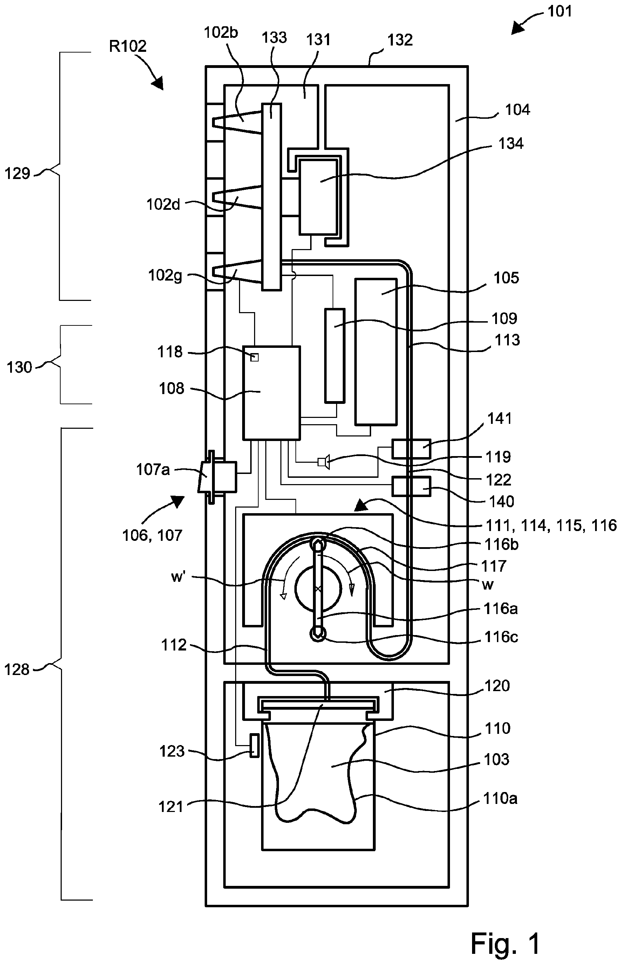

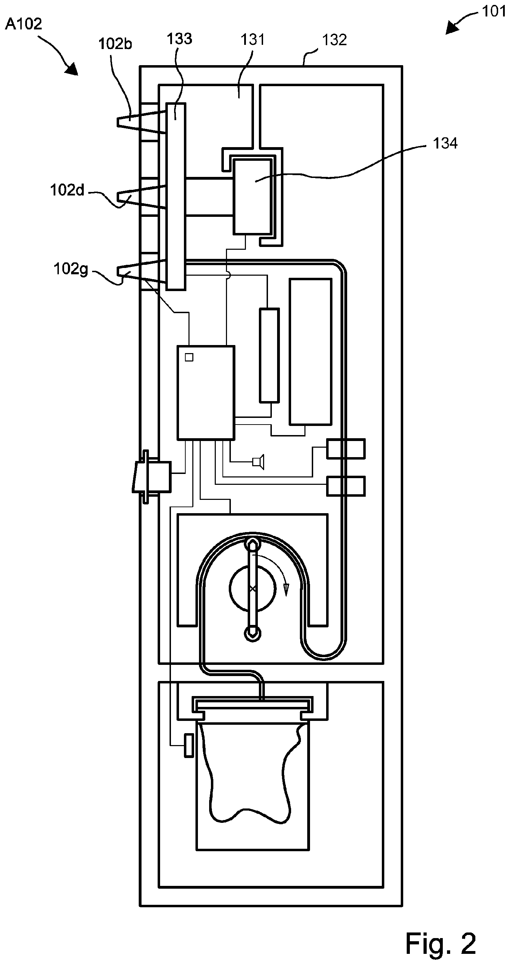

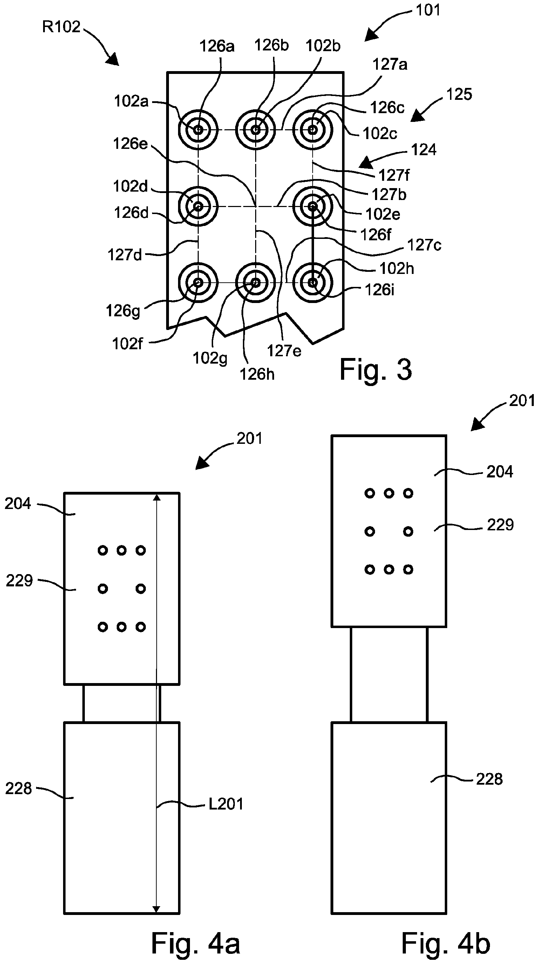

In der Figur 1 ist eine Schnittansicht durch eine schematische Darstellung einer ersten Ausführungsvariante eines Zerstäubers 101 gezeigt, wobei Zerstäuberdüsen 102a bis 102h in einer Ruhestellung R102 stehen. In der Figur 2 ist der in der Figur 1 gezeigte Zerstäuber 101 mit Zerstäuberdüsen 102a bis 102h gezeigt, welche in einer Arbeitsstellung A102 stehen. Die Figur 3 zeigt schließlich eine Ansicht auf den in der Figur 1 dargestellten Zerstäuber 101 als Ausschnitt im Bereich seiner Zerstäuberdüsen 102a bis 102h.

Der elektrostatische Zerstäuber 101 ist für eine elektrostatische Zerstäubung von Flüssigkeiten 103 vorgesehen. Hierbei werden unter Flüssigkeiten insbesondere Kosmetika im weiteren Sinne, aber z.B. auch Farben und Lacke verstanden. Der Zerstäuber 101 umfasst ein Gehäuse 104, eine elektrische Energiequelle 105, ein Aktivierungsmittel 106, welches als elektrischer Taster 107 ausgeführt ist, eine Kontrollelektronik 108, eine Hochspannungsquelle 109, einen Flüssigkeitstank 110, eine Fördervorrichtung 111 und die erwähnten Zerstäuberdüsen 102a bis 102h. Die Fördervorrichtung 111 ist zwischen dem Flüssigkeitstank 110 und den Zerstäuberdüsen 102a bis 102h angeordnet. Hierbei ist die Fördervorrichtung 111 durch eine erste Leitung 112 mit dem Flüssigkeitstank 110 verbunden ist und hierbei ist die Fördervorrichtung 111 durch eine zweite Leitung 113 mit den Zerstäuberdüsen 102a bis 102h verbunden, so dass die Fördervorrichtung 111 im Sprühbetrieb Flüssigkeit 103 aus dem Flüssigkeitstank 110 saugt und zu den Zerstäuberdüsen 102a bis 102h fördert. Die Leitung 112 sowie 113 ist vorliegend als einstückiger durchgehender Schlauch dargestellt. Aus Gründen der Übersichtlichkeit ist nur ein Schlauch zum Düsenfeld der Zerstäuberdüsen dargestellt. Die Zerstäuberdüsen sind aber durch mehrere Schläuche, nämlich einen Schlauch pro Zerstäuberdüse versorgt werden. Im Fall der Verwendung mehrerer Schläuche ist auch eine Mehrfach-Schlauchpumpe vorzusehen, welche z.B. durch einen verbreiterten Rotor mehrere Schläuche, angeordnet in der Art eines Flachbands (ähnlich eines Flachbandkabels) parallel pumpen kann.

Die Fördervorrichtung 111 ist als Pumpe 114 nämlich Verdrängerpumpe, insbesondere Peristaltikpumpe in der Bauart einer Saugpumpe 115 ausgebildet. Die Saugpumpe 115 ist als Schlauchpumpe 116 ausgebildet und so ausgeführt, dass ein zwischen der ersten Leitung 112 und der zweiten Leitung 113 angeordneten Förderschlauch 117 im Pumpbetrieb von an einem Rotor 116a angeordneten Rollen 116b, 116c abwechselnd wälzend deformiert und hierbei vollständig abdrückt wird, wie dies in der Figur 1 im Bereich der Rolle 116b gezeigt ist.

Der Zerstäuber 101 umfasst eine Umschaltvorrichtung 118, mittels welcher eine Drehrichtung des Rotors 116a der Schlauchpumpe 116 derart umschaltbar ist, dass die Schlauchpumpe zwischen einem Förderbetrieb, bei welchen sich der Rotor 116a in eine Drehrichtung w dreht und einem Rückförderbetrieb, bei welchem sich der Rotor 116a in eine entgegen gesetzte Drehrichtung w` dreht, wechselt. Durch einen kurzzeitigen Rückförderbetrieb ist es möglich, ein ungewünschtes Austreten von Flüssigkeit 103 nach dem Sprühen zu vermeiden. Hierbei ist es vorgesehen, eine Rückförderung der Flüssigkeit 103 in den Flüssigkeitstank 110 zu vermeiden. Dies wird dadurch erreicht, dass die erste Leitung 112, welche wie der Förderschlauch 117 elastisch dehnbar ausgebildet ist, durch das Rückfördern zur Vergrößerung ihres Innenvolumens leicht gedehnt wird und der Flüssigkeitstank 110 durch ein nicht dargestelltes Rückschlagventil verschlossen bleibt. Um für eine Rückförderung ein großes Speichervolumen der ersten Leitung sicherzustellen, ist es vorgesehen, die erste Leitung wenigstens 1,5 und insbesondere zweimal so lange auszubilden wie dies zur Verbindung des Flüssigkeitstanks mit der Fördervorrichtung erforderlich ist. Alternativ ist es auch vorgesehen, kleine Mengen von Flüssigkeit beim Rückförderbetrieb über eine Weiche bzw. ein entsprechendes Ventil in einen Entsorgungstank zu leiten. Hierdurch ist es möglich, die zweite Leitung 113 weitgehend vollständig zu entleeren.

Die Fördervorrichtung 101 und die Hochspannungsquelle 109 sind über das Aktivierungsmittel 106, welches durch den elektrischen Taster 107 gebildet ist, aktivierbar. Der Taster kann dabei aber auch als elektronischer Taster, insbesondere als kapazitiver Sensor, als Reed-Kontakt oder auch als Hall Sensor ausgebildet sein. Bei dem Aktivierungsmittel ist ein Betätigungselement 107a des Tasters 107 elektrisch leitfähig und als Gegenpol zu den Zerstäuberdüsen 102a bis 102b ausgebildet ist. Nachdem der Taster 107 zum Sprühen dauerhaft gedrückt sein muss, ist auf diese Weise eine gute Verbindung des Anwenders mit dem Bezugspotential der Sprühvorrichtung 101 sicher gestellt.

Der Zerstäuber 101 umfasst einen akustischen Signalgeber 119, welcher an die Kontrollelektronik 108 angeschlossen ist. Hierdurch kann der Anwender akustisch über eine korrekte oder fehlerhafte Anwendung des Zerstäubers 101 informiert werden und die Handhabung der Zerstäubers 101 damit trainiert und optimiert werden. Zusätzlich oder kumulativ ist auch ein vibrierender Signalgeber vorgesehen, wobei dieser mit einer bestimmten Frequenz immer dann aktiv ist, wenn der Zerstäuber sprüht. Durch ein Vibrieren mit einer abweichenden Frequenz können dem Anwender weitere Informationen vermittelt werden.

Der Zerstäuber 101 umfasst ein Anschlussmittel 120, wobei der Flüssigkeitstank 110 lösbar und über ein selbst schließendes Ventil 121, mit dem Anschlussmittel 120 verbunden ist. Hierdurch ist ein Kuppeln und Entkuppeln des Flüssigkeitstanks 110 mit einfachen Handbewegungen möglich und hierdurch ist auch einem ungewünschten Auslaufen des Flüssigkeitstanks 110 wirksam vorgebeugt.

Der Flüssigkeitstank 110 ist als selbst kollabierender Tank ausgebildet ist und umfasst hierzu einen Folienbeutel 110a. Derartige Flüssigkeitstanks lassen sich kostengünstig herstellen. Weiterhin ist der Füllstand derartiger Flüssigkeitstanks durch eine Sichtprüfung bei entsprechender Verwendung von transparenten Bauteilen und/oder entsprechender Positionierung von Öffnungen leicht feststellbar.

Die erste Leitung 112, die zweite Leitung 113 und der Förderschlauch 117 sind einteilig ausgebildet und durch einen Verbindungsschlauch 122 gebildet. Hierdurch ist die Zahl der Verbindungsstellen reduziert, so dass die Gefahr eine Ausbildung von Ablagerungen reduziert ist.

Zwischen dem Flüssigkeitstank 110 und der Kontrollelektronik 108 sind Mittel 123 zur Identifizierung des Flüssigkeitstanks 110 umfasst, wobei diese Mittel 123 als Erfassungsmittel ausgebildet sind, welche auf dem Flüssigkeitstank aufgedruckte bzw. an dem Flüssigkeitstank gespeicherte Informationen erfassen und der Kontrollelektronik entsprechende Informationen weiterleiten. Hierdurch ist die Kontrollelektronik 108 in der Lage verschiedene Parameter wie Höhe der Hochspannung, Leistung der Pumpe usw. auf die zu versprühende Flüssigkeit 103 anzupassen. Die Identifikationsmittel können insbesondere als RFID Markierungen ausgebildet sein, welche von der Kontrollelektronik 108 in dem Zerstäuber gelesen werden können. Besonders bevorzug ist es, dass die Kontrollelektronik 108 die Markierungen, insbesondere die RFID Markierungen auch beschreiben kann. Dadurch kann z.B. ein Datum vermerkt werden, an welchem ein Flüssigkeitstank zum ersten Mal in Benutzung genommen wurde oder welche Menge bereits entnommen ist.

Der Zerstäuber 101 umfasst 8 Zerstäuberdüsen 102a bis 102h, welche ringartig angeordnet sind (siehe Figur 3). Hierdurch lassen sich Körperflächen von einigen Quadratzentimetern besprühen, so dass ein rascher Sprühfortschritt gewährleistet ist.

Die Zerstäuberdüsen 102a bis 102h bilden ein Düsenfeld 124 (siehe Figur 3), wobei das Düsenfeld 124 als Gitter 125 mit sich an Gitterpunkten 126a bis 126i kreuzenden Gitterlinien 127a bis 127f ausgebildet ist, wobei es insbesondere vorgesehen ist, dass die Zerstäuberdüsen 102a bis 102h Gitterpunkte paralleler Gitterlinien bilden. Durch eine derartige regelmäßige geometrische Anordnung lässt sich ein gleichmäßiger Auftrage der Flüssigkeit erreichen.

Aus den Figuren 1 und 2 ist wieder ersichtlich, dass das Gehäuse 101 einen Griffabschnitt 128, einen Kopfabschnitt 129 und einen zwischen dem Griffabschnitt 128 und dem Kopfabschnitt 129 angeordneten Mittelabschnitt 130 umfasst, wobei die Zerstäuberdüsen 102a bis 102h in dem Kopfabschnitt 129 angeordnet sind, wobei die Fördervorrichtung 111 zwischen dem Flüssigkeitstank 110 und den Zerstäuberdüsen 102a bis 102h angeordnet ist.

Wie aus der Figur 1 ersichtlich ist, ist es auch vorgesehen, dass der Zerstäuber 101 eine Heizvorrichtung 140 und eine Kühlvorrichtung 141 umfasst, wobei die Flüssigkeit 103 vor dem Austritt aus den Zerstäuberdüsen 102a bis 102h zur Erreichung einer vorgegebenen Temperatur und/oder Viskosität hierdurch geheizt oder gekühlt werden kann.

Alternativ ist es auch vorgesehen, den Flüssigkeitstank zwischen der Fördervorrichtung und den Zerstäuberdüsen anzuordnen.

Die Figuren 4a und 4b zeigen eine zweite Ausführungsvariante eines Zerstäubers. Bei dem Zerstäuber 201 ist ein Gehäuse 204 telekopierbar ausgeführt, so dass ein Abstand zwischen einem Kopfabschnitt 229 und einem Griffabschnitt 228 veränderbar ist (vergleiche Figuren 4a und 4b). Hierdurch ist einem Anwender beispielsweise das Einsprühen der Füße erleichtert.

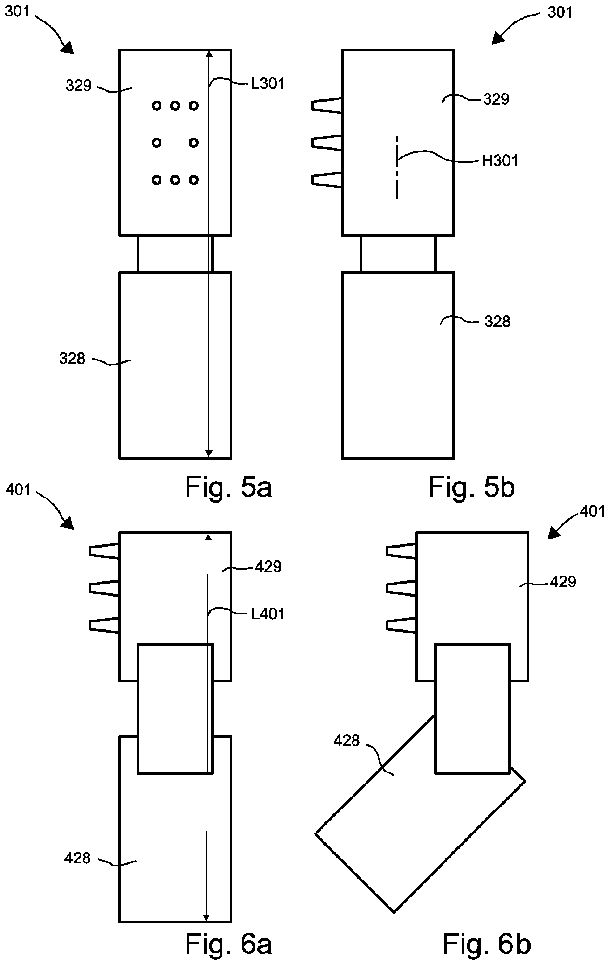

Die Figuren 5a und 5b zeigen eine dritte Ausführungsvariante eines Zerstäubers. Bei dem Zerstäuber 301 sind ein 329 Kopfabschnitt und ein Griffabschnitt 328 gegeneinander verdrehbar. In der Figur 5a ist der Zerstäuber 301 unverdreht gezeigt und in der Figur 5b ist der Kopfabschnitt 329 gegenüber dem Griffabschnitt 328, an welchem auch ein nicht dargestelltes Aktivierungsmittel angeordnet ist, um 90° um eine Hochachse H301 nach rechts verdreht. Durch eine derartige Verdrehbarkeit ist z.B. das Einsprühen der Schultern vereinfacht.

Die Figuren 6a und 6b zeigen eine vierte Ausführungsvariante eines Zerstäubers. Der Zerstäuber 401 weist einen Griffabschnitt 428 auf, welcher gegenüber einem Kopfabschnitt 429 dadurch abknickbar ist, dass der Zerstäuber 401 ein Knickgelenk umfasst. Hierdurch ist z.B. das Einsprühen der Fußsohlen oder des Rückens vereinfacht.

Selbstverständlich ist es auch vorgesehen, die drei in den Figuren 4a bis 6a gezeigten Bauformen zu kombinieren und so deren Vorteile zu vereinen, so dass ein Zerstäuber gebildet wird, welcher optimal für die Eigenanwendung geeignet ist.

Bei den Zerstäubern 201, 301 und 401 ist es vorgesehen, diese mit einer Länge L201 bzw. L301 bzw. L401 von etwa 10 cm bis 25 cm auszubilden, wobei die Länge L204 bei dem teleskopierbaren Zerstäuber 201 die Länge angibt, welche dieser im zusammengeschobenen Zustand (siehe Figur 4a) aufweist.

Gemäß einer nicht dargestellten Ausführungsvariante ist es auch vorgesehen, dass der Zerstäuber zusätzlich zu dem ersten Flüssigkeitstank einen zweiten Flüssigkeitstank umfasst, dass der Zerstäuber zusätzlich zu der ersten Fördervorrichtung eine zweite Fördervorrichtung umfasst und dass zwischen den Fördervorrichtungen und den Zerstäuberdüsen eine Mischvorrichtung zwischengeschaltet ist. Auf diese Weise lassen sich unterschiedliche Flüssigkeiten mischen. Durch eine unterschiedliche Ansteuerung der Fördereinrichtungen hinsichtlich der Drehzahl der Rotoren, lassen sich die beiden Flüssigkeiten auch mit mengenmäßig unterschiedlicher Zusammensetzung mischen, so dass beispielsweise der Anteil von Pflegestoffen erhöht oder erniedrigt werden kann.

Eine weitere nicht dargestellte Ausführungsvariante sieht vor, dass der Zerstäuber eine Heizvorrichtung und/oder eine Kühlvorrichtung umfasst, wobei die Flüssigkeit vor dem Austritt aus der wenigstens einen Zerstäuberdüse zur Erreichung einer vorgegebenen Temperatur und/oder Viskosität heizbar oder kühlbar ist. Hierdurch lassen sich bei unterschiedlichen Umgebungsbedingungen weitgehend vergleichbare Sprühergebnisse erzielen.

Es ist auch vorgesehen den Zerstäuber so zu betrieben, dass die Fördervorrichtung zur Förderung der Flüssigkeit sowohl in Vorschubrichtung, also zum Transport von Flüssigkeit vom Flüssigkeitstank hin zu den Zerstäuberdüsen, als auch in Rückzugsrichtung, also zum Transport von Flüssigkeit von der Zerstäuberdüse in Richtung des Flüssigkeitstanks betrieben wird. Durch einen derartigen Zweirichtungsbetrieb der Fördereinrichtung bringt diese den Zusatznutzen mit, dass ein Ausströmen von Flüssigkeit nach dem Sprühen zuverlässig vermieden werden kann.

Bei dem in den Figuren 1 bis 3 gezeigten Zerstäuber 101 ist - wie bereits erwähnt - eine Umschaltung zwischen einer Ruhestellung R102 und einer Arbeitsstellung A102 möglich. Hierbei sind die Zerstäuberdüsen 102a bis 102h in der Ruhestellung R102 (siehe Figur 1) in einem Innenraum 131 des Gehäuses 104 positioniert sind und ragen in der Arbeitsstellung A102 wenigstens abschnittsweise über eine Außenseite 132 des Gehäuses 104 hinaus (siehe Figur 2). Hierzu sind das Gehäuse 104 und die Zerstäuberdüsen 102a bis 102h zueinander beweglich, wobei die Zerstäuberdüsen 102a bis 102h auf einer Basisplatte 133 angeordnet sind und gemeinsam mit dieser von einem Hubzylinder 134 relativ zu dem Gehäuse 104 verfahr sind. Durch eine derartige Verfahrbarkeit sind die Zerstäuberdüsen 102a bis 102h bei Nichtgebrauch des Zerstäubers 101 vor Beschädigungen geschützt. Weiterhin wird hierdurch auch ein ungewünschtes Hängebleiben des Zerstäubers 101 z.B. beim Entnehmen aus einer Handtasche zuverlässig vermieden.

Alternativ oder kumulativ ist es auch vorgesehen die Zerstäuberdüsen durch Schieber, Klappen, Deckel und vergleichbare Abdeckmittel zu verdecken bzw. über eine Außenseite des Gehäuses hinaus stehen zu lassen. Insbesondere besteht die Möglichkeit, diese Schieber, Klappen oder Deckel mit Schaltern zu versehen, um z.B. eine Betriebsbereitschaft bei geöffnetem Schieber oder eine Abschaltung des Gerätes bei geschlossenem Schieber zu schalten.

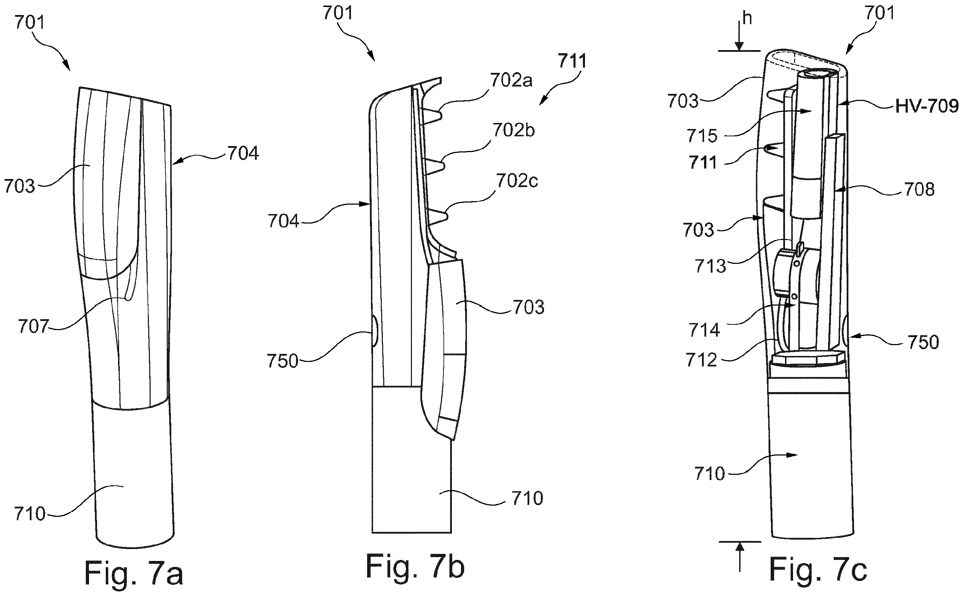

Figuren 7a bis c zeigt diesbezüglich eine weitere Ausführungsvariante 701 des erfindungsgemäßen Zerstäubers. Figur 7a zeigt dabei den Zerstäuber 701 mit einem Gehäuse 704. Am unteren Ende des Gehäuses 704 ist ein Flüssigkeitstank 710 angeordnet, welcher vorliegend als Tank mit passivem Folgekolben (nicht dargestellt) ausgebildet ist. Am oberen Ende weist das Gehäuse 704 einen Schieber 703 auf, welcher in Fig. 7a geschlossen dargestellt ist. Durch ein Verschieben des Schiebers 703 entlang einer Führung 707 in Richtung des unteren Endes des Gehäuses 704 wird der Schieber 703 in die in Fig. 7b dargestellte Position gebracht. Durch dieses Verschieben des Schiebers 703 wird ein Düsenfeld 711 mit drei Zerstäuberdüsen 702a, 702b und 702c freigegeben. Der Schieber verfügt dabei über Schaltmittel, welche erst bei geöffnetem Schieber 703, also in der Position entsprechend Fig. 7b, eine Betriebsbereitschaft des Zerstäubers 701 herstellen und die notwendigen Komponenten an der Kontrollelektronik 708 freigeben.

Das Düsenfeld 711 umfasst vorliegend drei Zerstäuberdüsen 702a, 702b, 702c, welche in der dargestellten Fassung aus Silikon gefertigt sind. Erfindungsgemäß sind auch andere Materialien, insbesondere auch harte Kunststoffe wie z.B. PE oder PP denkbar, welche sich für die elektrostatische Wechselwirkung zur Zerstäubung eignen. Durch die für die Zerstäubung genutzte Hochspannung aus der Hochspannungsquelle 709 kann insbesondere bei Silikondüsen ein gutes Zerstäubungsergebnis erzielt werden.

In Fig 7b ist auf der dem Schieber 703 gegenüber liegenden Seite ein elektrischer Taster 750 vorgesehen. Dieser Taster dient zur Aktivierung des Sprühvorgangs. Neben einem einfachen elektrischen Taster kann auch ein elektronischer Schalter z.B. durch eine kapazitive oder ähnliche Schaltauslösung vorgesehen sein. Eine Kontaktfläche am Taster dient zur elektrischen Kontaktierung des Benutzers zur Vermeidung von Aufladungen durch die elektrostatische Zerstäubung.

Fig. 7c zeigt die schematische Anordnung der Komponenten der dargestellten Ausführungsform des Zerstäubers 701. Oberhalb des Flüssigkeitstanks 710 ist eine Schlauchpumpe 714 angeordnet. Die Zerstäuberdüsen 702a, 702b und 702c sind über ein Schlauchpaket 713 von drei Schläuchen, wobei jede Zerstäuberdüse mit einem eigenen Schlauch verbunden ist, über die Schlauchpumpe 714 in Ausgestaltung einer Mehrkanalpumpe mit dem Flüssigkeitstank über ein Schlauchpaket 712 verbunden. Die Schlauchpakete sind dabei als nebeneinander liegende, einstückig extrudierte Schläuche, vergleichbar mit der Anordnung eines Flachbandkabels, ausgebildet.

Hinter der Schlauchpumpe 714 ist eine Kontrollelektronik 708 angeordnet. Auf der Rückseite des Gehäuses 704 ist auch in Fig. 7c der Taster 750 zur Bedienung des Zerstäubers und Kontaktierung der Bedienperson dargestellt. Oberhalb der Schlauchpumpe 714 ist eine Batterie 715 als Energiequelle angeordnet. Die Batterie 715 ist dabei raumsparend hinter dem Düsenfeld 711 untergebracht, wobei vorliegend in Fig. 7c der Schieber 703 nach oben geschoben und das Düsenfeld 711 abgedeckt ist. Die Gesamte Anordnung weißt eine Höhe h von ca. 27cm auf und ist damit handlich zu bedienen und gleichzeitig in der Lage, schwierige Stellen, z.B. am Rücken einer Bedienperson erreichbar zu machen.

### Bezugszeichenliste:

- 101: Zerstäuber (erste Variante)
- 102a - 102h: Zerstäuberdüse
- 103: Flüssigkeiten
- 104: Gehäuse
- 105: Energiequelle
- 106: Aktivierungsmittel
- 107: elektrischer Taster
- 107a: Betätigungselement von 107
- 108: Kontrollelektronik
- 109: Hochspannungsquelle
- 110: Flüssigkeitstank
- 110a: Folienbeutel
- 111: Fördervorrichtung
- 112: erste Leitung
- 113: zweite Leitung
- 114: Pumpe
- 115: Saugpumpe
- 116: Schlauchpumpe
- 117: Förderschlauch
- 116a: Rotor
- 116b, 116c: Rolle
- 117: Förderschlauch
- 118: Umschaltvorrichtung
- 119: Signalgeber
- 120: Anschlussmittel
- 121: Ventil
- 122: Verbindungsschlauch
- 123: Mittel zur Identifizierung des Flüssigkeitstanks
- 124: Düsenfeld
- 125: Gitter
- 126a - 126i: Gitterpunkt
- 127a - 127f: Gitterlinie
- 128: Griffabschnitt
- 129: Kopfabschnitt
- 130: Mittelabschnitt
- 131: Innenraum
- 132: Außenseite
- 133: Basisplatte
- 134: Hubzylinder

- 140: Heizvorrichtung
- 141: Kühlvorrichtung

- A102: Arbeitsstellung
- R102: Ruhestellung
- w, w`: Drehrichtung

- 201: Zerstäuber
- 204: Gehäuse
- 228: Griffabschnitt
- 229: Kopfabschnitt
- L201: Länge von 201

- 301: Zerstäuber
- 328: Griffabschnitt
- 329: Kopfabschnitt
- H301: Hochachse von 301
- L301: Länge von 301

- 401: Zerstäuber
- 428: Griffabschnitt
- 429: Kopfabschnitt
- L401: Länge von 401

- 701: Zerstäuber
- 702a - 702c: Zerstäuberdüsen
- 703: Schieber
- 704: Gehäuse
- 707: Führung
- 708: Kontrollelektronik
- 709: Hochspannungsquelle
- 710: Flüssigkeitstank
- 711: Düsenfeld
- 712: Schlauchpaket
- 713: Schlauchpaket
- 714: Schlauchpumpe
- 715: Batterie
- 750: Taster
- h: Höhe des Geräts

## Patentansprüche

1. Elektrostatischer Zerstäuber (101) für Flüssigkeiten (103), insbesondere Kosmetika,
wobei der Zerstäuber (101)
- ein Gehäuse (104),
- eine elektrische Energiequelle (105),
- ein Aktivierungsmittel (106),
eine Kontrollelektronik (108),
- eine Hochspannungsquelle (109),
- einen Flüssigkeitstank (110),
- eine Fördervorrichtung (111) und
- Zerstäuberdüsen (102a - 102h) umfasst,
- wobei die Fördervorrichtung (111) zwischen dem Flüssigkeitstank (110) und den Zerstäuberdüsen (102a - 102h) angeordnet ist,
- wobei die Fördervorrichtung (111) durch eine erste Leitung (112) mit dem Flüssigkeitstank (110) verbunden ist und
- wobei die Fördervorrichtung (111) Flüssigkeit (103) aus dem Flüssigkeitstank (110) saugt und zu den Zerstäuberdüsen (102a - 102h) fördert,
**dadurch gekennzeichnet,**
- **dass** die Fördervorrichtung (111) durch eine zweite Leitung mit (113) den Zerstäuberdüsen (102a - 102h) verbunden ist,
- **dass** die erste Leitung (112), die zweite Leitung (113) und ein Förderschlauch (117) einteilig ausgebildet sind und als Verbindungsschlauch (122) ausgebildet sind
- **dass** die erste Leitung (112) sowie die zweite Leitung (113) mehrere Schläuche umfasst und pro Zerstäuberdüse (102a - 102h) jeweils ein Schlauch von der Düse durch die Fördereinrichtung zum Flüssigkeitstank (110) führt.

2. Zerstäuber nach Anspruch 1, **dadurch gekennzeichnet, dass** die Fördervorrichtung (111) als Pumpe (114) und insbesondere als Verdrängerpumpe, bevorzugt als Schlauchpumpe, insbesondere für mehrere Schläuche, z.B. als Mehrkanal-Peristaltikpumpe, und vorzugsweise als Saugpumpe (115) zum Ansaugen von Flüssigkeit (103) aus dem Flüssigkeitstank (110) ausgebildet ist, wobei es insbesondere vorgesehen ist, dass die Schlauchpumpe (116) einen zwischen der ersten Leitung (112) und der zweiten Leitung (113) angeordneten Förderschlauchabschnittt (117) im Pumpbetrieb wälzend deformiert und hierbei insbesondere vollständig abdrückt.

3. Zerstäuber nach einem der vorangegangenen Ansprüche, **dadurch gekennzeichnet, dass** der Zerstäuber (101) eine Umschaltvorrichtung (118) umfasst, mittel welcher eine Förderrichtung der Fördervorrichtung, insbesondere eine Drehrichtung (w, w') eines Rotors (116a) einer Schlauchpumpe (116) derart umschaltbar ist, dass die Schlauchpumpe (116) zwischen einem Förderbetrieb, z.B. als Saugpumpe auf der Seite des Flüssigkeitstanks und einem Rückförderbetrieb, z.B. als Druckpumpe auf der Seite des Flüssigkeitstanks umschaltbar ist.

4. Zerstäuber nach einem der vorangegangenen Ansprüche, **dadurch gekennzeichnet, dass** die Fördervorrichtung (111) und/oder die Hochspannungsquelle (109) über ein Aktivierungsmittel (106), insbesondere einen Schalter oder einen Taster (107) aktivierbar ist bzw. sind, wobei ein Betätigungselement (107a) des Schalters oder des Tasters (107) elektrisch leitfähig und insbesondere als Gegenpol zu den Zerstäuberdüsen (102a - 102h) ausgebildet ist und/oder dass der Zerstäuber über einen Hauptschalter zur Freigabe eines Betriebszustandes verfügt, wobei der Hauptschalter insbesondere als Schieber, Schiebeschalter, Taster oder Sensor ausgebildet ist.

5. Zerstäuber nach einem der vorangegangenen Ansprüche, **dadurch gekennzeichnet, dass** der Zerstäuber (101) einen akustischen Signalgeber (119) und/oder einen optischen Signalgeber und/oder einen haptischen, insbesondere vibrierenden Signalgeber umfasst, welcher an die Kontrollelektronik (108) angeschlossen ist.

6. Zerstäuber nach einem der vorangegangenen Ansprüche, **dadurch gekennzeichnet, dass** der Zerstäuber (101) ein Anschlussmittel (120) umfasst, wobei der Flüssigkeitstank (110) lösbar, insbesondere über ein selbst öffnendes sowie selbst schließendes Ventil (121), mit dem Anschlussmittel (120) verbunden ist und/oder der Flüssigkeitstank das Anschlussmittel umfasst.

7. Zerstäuber nach einem der vorangegangenen Ansprüche, **dadurch gekennzeichnet, dass** der Flüssigkeitstank (110) als selbst kollabierender Tank ausgebildet ist und insbesondere einen Folienbeutel (110a) oder einen Behälter mit passiv folgendem Kolben umfasst.

8. Zerstäuber nach einem der vorangegangenen Ansprüche, **dadurch gekennzeichnet, dass** die erste Leitung (112), die zweite Leitung (113) und der Förderschlauch (117) als extrudierter Kunststoffschlauch, bevorzug als Gummischlauch ausgebildet sind.

9. Zerstäuber nach einem der vorangegangenen Ansprüche, **dadurch gekennzeichnet, dass** die erste Leitung sowie die zweite Leitung einstückig extrudiert, als Schlauchpaket mit mehreren parallel verlaufenden Schläuchen, ausgebildet ist.

10. Zerstäuber nach einem der vorangegangenen Ansprüche, **dadurch gekennzeichnet, dass** zwischen dem Flüssigkeitstank (110) und der Kontrollelektronik (108) und/oder dem Gehäuse (104) zur Aufnahme des Flüssigkeitstanks (110) Mittel (123) zur Identifizierung des Flüssigkeitstanks (110) umfasst sind, wobei diese Mittel (123) insbesondere als mechanische Kodierung, bevorzugt unter Ausbildung eines Formschlusses, und/oder elektronische Kodierung, z.B. als RFID Kodierung, insbesondere unter Übermittlung von Informationen über den Flüssigkeitstank, und/oder elektrische Kodierung, insbesondere durch Ausbildung von elektrischen Leitungswegen über Kontakte, ausgebildet sind.

11. Zerstäuber nach einem der vorangegangenen Ansprüche, **dadurch gekennzeichnet, dass** der Zerstäuber (101) wenigstens 8 und insbesondere wenigstens 20 Zerstäuberdüsen (102a - 102h) umfasst.

12. Zerstäuber nach einem der vorangegangenen Ansprüche, **dadurch gekennzeichnet, dass** der Zerstäuber (101) wenigstens zwei, bevorzugt drei und insbesondere höchstens 8 Zerstäuberdüsen umfasst.

13. Zerstäuber nach einem der vorangegangenen Ansprüche, **dadurch gekennzeichnet, dass** die Zerstäuberdüsen (102a - 102h) ein Düsenfeld (124) bilden, wobei das Düsenfeld (124) als Gitter (125) mit sich an Gitterpunkten (126a - 126i) kreuzenden Gitterlinien (127a - 127f) ausgebildet ist, wobei es insbesondere vorgesehen ist, dass die Zerstäuberdüsen (102a - 102h) Gitterpunkte (126a - 126i) paralleler Gitterlinien (127a, 127b, 127c; 127d, 127e, 127f) oder Eckpunkte einer dreieckigen Gitterstruktur bilden.

14. Zerstäuber nach einem der vorangegangenen Ansprüche, **dadurch gekennzeichnet, dass** das Gehäuse (104) einen Griffabschnitt (128), einen Kopfabschnitt (129) und einen zwischen dem Griffabschnitt (128) und dem Kopfabschnitt (129) angeordneten Mittelabschnitt (130) umfasst, wobei die Zerstäuberdüsen (102a - 102h) in dem Kopfabschnitt (129) angeordnet sind, wobei die Fördervorrichtung (111) zwischen dem Flüssigkeitstank (110) und den Zerstäuberdüsen (102a - 102h) angeordnet ist, und/oder dass das Gehäuse (104) des Zerstäubers (101; 201; 301; 401) eine Länge (L101; L201; L301; L401) von etwa 10 cm bis 30 cm, insbesondere ca. 27cm aufweist.

15. Zerstäuber nach einem der vorangegangenen Ansprüche, **dadurch gekennzeichnet, dass** das Gehäuse (201; 301; 401) telekopierbar ist und hierdurch ein Abstand zwischen dem Kopfabschnitt (229) und dem Griffabschnitt (228) veränderbar ist und/oder dass der Kopfabschnitt (329) und der Griffabschnitt (328) gegeneinander verdrehbar sind und/oder dass der Kopfabschnitt (429) und der Griffabschnitt (428) in ihrem Winkel, welchen diese zueinander einnehmen, durch Knicken veränderbar sind.

16. Zerstäuber nach einem der vorangegangenen Ansprüche, **dadurch gekennzeichnet, dass** der Zerstäuber (101) zusätzlich zu dem ersten Flüssigkeitstank (110) einen zweiten Flüssigkeitstank umfasst, und/oder dass der Zerstäuber (101) zusätzlich zu der ersten Fördervorrichtung (111) eine zweite Fördervorrichtung umfasst, und/oder dass zwischen den Fördervorrichtungen und den Zerstäuberdüsen (102a - 102h) eine Mischvorrichtung zwischengeschaltet ist.

17. Zerstäuber nach einem der vorangegangenen Ansprüche, **dadurch gekennzeichnet, dass** der Zerstäuber (101) eine Heizvorrichtung (140) und/oder eine Kühlvorrichtung (141) umfasst, wobei die Flüssigkeit (103) vor dem Austritt aus den Zerstäuberdüsen (102a - 102f) zur Erreichung einer vorgegebenen Temperatur und/oder Viskosität heizbar oder kühlbar ist.

18. Verfahren zum Betrieb eines Zerstäubers (101) nach einem der vorangegangenen Ansprüche, **dadurch gekennzeichnet, dass** die Fördervorrichtung (111) zur Förderung der Flüssigkeit (103) sowohl in Vorschubrichtung, also zum Transport von Flüssigkeit (103) vom Flüssigkeitstank (110) hin zu den Zerstäuberdüsen (102a - 102h), als auch in Rückzugsrichtung, also zum Transport von Flüssigkeit (103) von der Zerstäuberdüse (102a - 102h) in Richtung des Flüssigkeitstanks (110) betrieben wird.

## Claims

1. Electrostatic atomizer (101) for liquids (103), especially cosmetics,
wherein the atomizer (101) comprises
- a housing (104),
- an electrical power source (105),
- activating means (106),
- a control electronics (108),
- a high voltage source (109),
- a liquid tank (110),
- a conveying device (111) and
- atomizer nozzles (102a - 102h),
- wherein the delivery device (111) is arranged between the liquid tank (110) and the atomizer nozzles (102a - 102h),
- wherein the delivery device (111) is connected to the liquid tank (110) by a first conduit (112), and
- wherein the delivery device (111) draws liquid (103) from the liquid tank (110) and delivers it to the atomizer nozzles (102a - 102h),
**characterized in that**
- the conveying device (111) is connected to (113) the atomizer nozzles (102a - 102h) by a second conduit,
- the first conduit (112), the second conduit (113) and a delivery hose (117) are formed in one piece and are formed as a connecting hose (122)
- the first conduit (112) as well as the second conduit (113) comprises several hoses and one hose per atomizer nozzle (102a - 102h) leads from the nozzle through the conveying device to the liquid tank (110).

2. Atomizer according to claim 1, **characterized in that** the delivery device (111) is designed as a pump (114) and in particular as a positive displacement pump, preferably as a peristaltic pump, in particular for several tubes, e.g. as a multi-channel peristaltic pump, and preferably as a suction pump (115) for drawing liquid (103) from the liquid tank (110), it being provided in particular that the peristaltic pump (116) deforms a delivery hose section (117) arranged between the first conduit (112) and the second conduit (113) in a rolling manner during pumping operation and in particular presses it off completely in the process.

3. Atomizer according to one of the preceding claims,
**characterized in that** the atomizer (101) comprises a changeover device (118) by means of which a conveying direction of the conveying device, in particular a direction of rotation (w, w') of a rotor (116a) of a peristaltic pump (116), can be changed over in such a way that the peristaltic pump (116) can be switched over between a conveying mode, for example as a suction pump on the side of the liquid tank, and a return conveying mode, for example as a pressure pump on the side of the liquid tank.

4. Atomizer according to one of the preceding claims,
**characterized in that** the conveying device (111) and/or the high-voltage source (109) can be activated via an activating means (106), in particular a switch or a pushbutton (107) wherein an actuating element (107a) of the switch or the pushbutton (107) is electrically conductive and is formed in particular as a counter pole to the atomizer nozzles (102a - 102h) and/or **in that** the atomizer has a main switch for releasing an operating state, wherein the main switch is formed in particular as a slide, slide switch, pushbutton or sensor.

5. Atomizer according to one of the preceding claims,
**characterized in that** the atomizer (101) comprises an acoustic signal transmitter (119) and/or an optical signal transmitter and/or a haptic, in particular vibrating signal transmitter, which is connected to the control electronics (108).

6. Atomizer according to one of the preceding claims,
**characterized in that** the atomizer (101) comprises a connection means (120), wherein the liquid tank (110) is detachably connected to the connection means (120), in particular via a self-opening as well as self-closing valve (121), and/or the liquid tank comprises the connection means.

7. Atomizer according to one of the preceding claims,
**characterized in that** the liquid tank (110) is designed as a self-collapsing tank and in particular comprises a foil bag (110a) or a container with a passively following piston.

8. Atomizer according to one of the preceding claims,
**characterized in that** the first conduit (112), the second conduit (113) and the delivery hose (117) are formed as extruded plastic hose, preferably as rubber hose.

9. Atomizer according to one of the preceding claims,
**characterized in that** the first conduit as well as the second conduit is extruded in one piece, as a hose package with several hoses running in parallel.

10. Atomizer according to one of the preceding claims,
**characterized in that** means (123) for identifying the liquid tank (110) are comprised between the liquid tank (110) and the control electronics (108) and/or the housing (104) for accommodating the liquid tank (110), wherein these means (123) are formed in particular as mechanical coding, preferably by forming a form fit, and/or electronic coding, e.g. as RFID coding, in particular by transmitting information about the liquid tank, and/or electrical coding, in particular by forming electrical conduction paths via contacts.

11. Atomizer according to one of the preceding claims,
**characterized in that** the atomizer (101) comprises at least 8 and in particular at least 20 atomizer nozzles (102a - 102h).

12. Atomizer according to one of the preceding claims,
**characterized in that** the atomizer (101) comprises at least two, preferably three, and in particular at most 8 atomizer nozzles.

13. Atomizer according to one of the preceding claims,
**characterized in that** the atomizer nozzles (102a - 102h) form a nozzle field (124), the nozzle field (124) being designed as a grid (125) with grid lines (127a - 127f) intersecting at grid points (126a - 126i), wherein in particular it being provided that the atomizer nozzles (102a - 102h) form grid points (126a - 126i) of parallel grid lines (127a, 127b, 127c; 127d, 127e, 127f) or corner points of a triangular grid structure.

14. Atomizer according to one of the preceding claims,
**characterized in that** the housing (104) comprises a handle portion (128), a head portion (129) and a middle portion (130) arranged between the handle portion (128) and the head portion (129), wherein the atomizer nozzles (102a - 102h) are arranged in the head portion (129), wherein the conveying device (111) is arranged between the liquid tank (110) and the atomizer nozzles (102a - 102h), and/or the housing (104) of the atomizer (101; 201; 301; 401) has a length (L101; L201; L301; L401) of about 10 cm to 30 cm, in particular approx. 27cm.

15. Atomizer according to one of the preceding claims,
**characterized in that** the housing (201; 301; 401) is telecopable and thereby a distance between the head portion (229) and the handle portion (228) is variable and/or that the head portion (329) and the handle portion (328) are rotatable relative to each other and/or that the head portion (429) and the handle portion (428) are variable in their angle, which they assume relative to each other, by buckling.

16. Atomizer according to one of the preceding claims,
**characterized in that** the atomizer (101) comprises, in addition to the first liquid tank (110), a second liquid tank, and/or that the atomizer (101) comprises, in addition to the first conveying device (111), a second conveying device, and/or that a mixing device is interposed between the conveying devices and the atomizer nozzles (102a - 102h).

17. Atomizer according to one of the preceding claims,
**characterized in that** the atomizer (101) comprises a heating device (140) and/or a cooling device (141), wherein the liquid (103) can be heated or cooled to achieve a predetermined temperature and/or viscosity before exiting the atomizer nozzles (102a - 102f).

18. Method of operating an atomizer (101) according to one of the preceding claims, **characterized in that** the conveying device (111) to convey the liquid (103) is operated both in the advancing direction, thus to transport liquid (103) from the liquid tank (110) towards the atomizer nozzles (102a - 102h), and in the retracting direction, thus to transport liquid (103) from the atomizer nozzle (102a - 102h) towards the liquid tank (110).

## Revendications

1. Pulvérisateur électrostatique (101) pour liquides (103), en particulier pour cosmétiques
dans lequel le pulvérisateur (101) comprend
- un boîtier (104),
- une source d'énergie électrique (105),
- un moyen d'activation (106),
- une électronique de contrôle (108),
- une source de haute tension (109),
- un réservoir de liquide (110),
- un dispositif de transport (111) et
- buses de pulvérisation (102a - 102h),
- dans lequel le dispositif de transport (111) est disposé entre le réservoir de liquide (110) et les buses de pulvérisation (102a - 102h),
- dans lequel le dispositif de transport (111) est relié au réservoir de liquide (110) par un premier conduit (112) et
- dans lequel le dispositif de transport (111) aspire le liquide (103) du réservoir de liquide (110) et le transporte vers les buses d'atomisation (102a - 102h),
**caractérisé en ce**
- **que** le dispositif de transport (111) est relié par une deuxième conduite aux buses de pulvérisation (102a - 102h),
- **que** la première conduite (112), la deuxième conduite (113) et un tuyau de transport (117) sont formés d'une seule pièce et sont conçus comme un tuyau de liaison (122)
- **que** la première conduite (112) ainsi que la deuxième conduite (113) comprennent plusieurs tuyaux et que, pour chaque buse de pulvérisation (102a - 102h), un tuyau conduit de la buse au réservoir de liquide (110) à travers le dispositif de transport.

2. Pulvérisateur selon la revendication 1, **caractérisé en ce que** le dispositif de transport (111) est formé comme une pompe (114) et en particulier comme une pompe volumétrique, de préférence comme une pompe péristaltique, en particulier pour plusieurs tuyaux, par ex. Comme une pompe péristaltique multicanaux, et de préférence comme une pompe d'aspiration (115) pour aspirer le liquide (103) du réservoir de liquide (110), il est notamment prévu que la pompe péristaltique (116) déforme par roulement un tronçon de tuyau de refoulement (117) disposé entre la première conduite (112) et la deuxième conduite (113) en mode de pompage et le presse en particulier complètement à cette occasion.

3. Pulvérisateur selon l'une des revendications précédentes, **caractérisé en ce que** lepulvérisateur (101) comprend un dispositif de commutation (118) au moyen duquel un sens de transport du dispositif de transport, en particulier un sens de rotation (w, w') d'un rotor (116a) d'une pompe péristaltique (116) peut être commuté de telle sorte que la pompe péristaltique (116) peut être commutée entre un mode de transport, par exemple comme pompe d'aspiration du côté du réservoir de liquide, et un mode de refoulement, par exemple comme pompe de refoulement du côté du réservoir de liquide.

4. Pulvérisateur selon l'une des revendications précédentes, **caractérisé en ce que** le dispositif de transport (111) et/ou la source de haute tension (109) est ou sont activables par un moyen d'activation (106), en particulier un interrupteur ou un bouton-poussoir (107), un élément d'actionnement (107a) de l'interrupteur ou du bouton-poussoir (107) étant électriquement conducteur et étant formé en particulier comme pôle opposé aux buses de pulvérisation (102a - 102h) et/ou **en ce que** le pulvérisateur dispose d'un interrupteur principal pour la libération d'un état de fonctionnement, l'interrupteur principal étant formé en particulier comme un coulisseau, un interrupteur coulissant, un bouton-poussoir ou un capteur.

5. Pulvérisateur selon l'une des revendications précédentes, **caractérisé en ce que** lepulvérisateur (101) comprend un générateur de signaux acoustiques (119) et/ou un générateur de signaux optiques et/ou un générateur de signaux haptiques, notamment vibratoires, qui est raccordé à l'électronique de contrôle (108).

6. Pulvérisateur selon l'une quelconque des revendications précédentes, **caractérisé en ce que** lepulvérisateur (101) comprend un moyen de raccordement (120), le réservoir de liquide (110) étant relié de manière amovible au moyen de raccordement (120), en particulier par l'intermédiaire d'une soupape (121) à ouverture automatique ainsi qu'à fermeture automatique, et/ou le réservoir de liquide comprenant le moyen de raccordement.

7. Pulvérisateur selon l'une quelconque des revendications précédentes, **caractérisé en ce que** le réservoir de liquide (110) est formé comme un réservoir à effondrement automatique et comprend notamment un sac en feuille (110a) ou un récipient à piston à suivi passif.

8. Pulvérisateur selon l'une des revendications précédentes, **caractérisé en ce que** le premier conduit (112), le deuxième conduit (113) et le tuyau de transport (117) sont réalisés sous la forme d'un tuyau en plastique extrudé, de préférence un tuyau en caoutchouc.

9. Pulvérisateur selon l'une des revendications précédentes, **caractérisé en ce que** le premier conduit ainsi que le deuxième conduit sont extrudés d'un seul tenant, sous la forme d'un paquet de tuyaux avec plusieurs tuyaux s'étendant parallèlement.

10. Pulvérisateur selon l'une des revendications précédentes, **caractérisé en ce qu'**entre le réservoir de liquide (110) et l'électronique de contrôle (108) et/ou le boîtier (104) destiné à recevoir le réservoir de liquide (110) sont compris des moyens (123) d'identification du réservoir de liquide (110), ces moyens (123) étant réalisés en particulier sous forme de codage mécanique, de préférence en formant une liaison par complémentarité de forme, et/ou de codage électronique, par exemple sous forme de codage RFID, en particulier en transmettant des informations sur le réservoir de liquide, et/ou de codage électrique, en particulier en formant des voies de conduction électrique par l'intermédiaire de contacts.

11. - Pulvérisateur selon l'une quelconque des revendications précédentes, **caractérisé en ce que** lepulvérisateur (101) comprend au moins huit et notamment au moins vingt buses de pulvérisation (102a - 102h).

12. Pulvérisateur selon l'une quelconque des revendications précédentes, **caractérisé en ce que** lepulvérisateur (101) comprend au moins deux, de préférence trois et notamment au plus huit buses de pulvérisation .

13. Pulvérisateur selon l'une des revendications précédentes, **caractérisé en ce que** les buses de pulvérisation (102a - 102h) forment un champ de buses (124), le champ de buses (124) étant réalisé sous forme de grille (125) avec des lignes de grille (127a - 127f) se croisant en des points de grille (126a - 126i), il est notamment prévu que les buses de pulvérisation (102a - 102h) forment des points de grille (126a - 126i) de lignes de grille parallèles (127a, 127b, 127c ; 127d, 127e, 127f) ou des points d'angle d'une structure de grille triangulaire.

14. Pulvérisateur selon l'une quelconque des revendications précédentes, **caractérisé en ce que** le boîtier (104) comprend une partie de poignée (128), une partie de tête (129) et une partie centrale (130) disposée entre la partie de poignée (128) et la partie de tête (129), les buses de pulvérisation (102a - 102h) étant disposées dans la partie de tête (129), le dispositif de transport (111) étant disposé entre le réservoir de liquide (110) et les buses d'atomisation (102a - 102h), et/ou **en ce que** le boîtier (104) dupulvérisateur (101 ; 201 ; 301 ; 401) a une longueur (L101 ; L201 ; L301 ; L401) d'environ 10 cm à 30 cm, notamment d'environ 27 cm.

15. Pulvérisateur selon l'une des revendications précédentes, **caractérisé en ce que** le boîtier (201 ; 301 ; 401) est téléscopique et que, de ce fait, une distance entre la section de tête (229) et la section de poignée (228) peut être modifiée et/ou **en ce que** la section de tête (329) et la section de poignée (328) peuvent être tournées l'une par rapport à l'autre et/ou **en ce que** la section de tête (429) et la section de poignée (428) peuvent être modifiées par pliage dans l'angle qu'elles forment l'une par rapport à l'autre.

16. Pulvérisateur selon l'une des revendications précédentes, **caractérisé en ce que** lepulvérisateur (101) comprend, en plus du premier réservoir de liquide (110), un deuxième réservoir de liquide, et/ou **en ce que** lepulvérisateur (101) comprend, en plus du premier dispositif de transport (111), un deuxième dispositif de transport, et/ou **en ce qu'**un dispositif de mélange est intercalé entre les dispositifs de transport et les buses de pulvérisation (102a - 102h).

17. - Pulvérisateur selon l'une des revendications précédentes, **caractérisé en ce que** lepulvérisateur (101) comprend un dispositif de chauffage (140) et/ou un dispositif de refroidissement (141), le liquide (103) pouvant être chauffé ou refroidi avant de sortir des buses de pulvérisation (102a - 102f) pour atteindre une température et/ou une viscosité prédéterminée.

18. Procédé de fonctionnement d'un pulvérisateur (101) selon l'une des revendications précédentes, **caractérisé en ce que** le dispositif de transport (111) est utilisé pour transporter le liquide (103) autantdans le sens de l'avancement, c'est-à-dire pour transporter le liquide (103) du réservoir de liquide (110) vers les buses de pulvérisation (102a-102h), que dans le sens du retrait, c'est-à-dire pour transporter le liquide (103) de la buse de pulvérisation (102a-102h) vers le réservoir de liquide (110).
